# EUROPEAN PATENT SPECIFICATION

(11) **EP 4 536 975 B1**
(45) Date of publication and mention of the grant of the patent: **19.08.2026**
(21) Application number: 23735953.4
(22) Date of filing: 09.06.2023
(51) Int. Cl.: F04C 18/16, F04C 2/107

(54) **SCREW PUMP AND ITS COMPONENTS**
SCHRAUBENPUMPE UND IHRE KOMPONENTEN
POMPE À VIS ET SES COMPOSANTS

(30) Priority: 10.06.2022 FR 2205592
(43) Date of publication of application: 16.04.2025
(73) Proprietor: Illinois Tool Works, Inc., Glenview, Illinois 60025 (US)
(72) Inventor: MARTIN, Lionel, Glenview, Illinois 60025 (US); RICHARD, Didier, Glenview, Illinois 60025 (US)
(74) Representative: HGF
(86) International application number: PCT/US2023/068205
(87) International publication number: WO 2023/240238

(56) References cited:
- EP-A1- 3 816 419
- EP-A1- 3 816 446
- WO-A1-86/05555
- FR-A1- 2 429 909
- GB-A- 2 076 471
- US-A- 3 461 949

## Description

### Technical Field

The invention relates to the field of screw pumps and their components. More specifically, but not exclusively, this disclosure also relates to a cooling circuit, for example for a vehicle, that comprises the screw pump.

### Technical Background

Known screw pumps comprise a casing and two, three or more screws housed in the casing, which are driven by a motor to force fluid flow through the pump. FR 2429909A describes an externally-meshing screw pump comprising a main screw which meshes with idler screws. EP 3816419A1 and EP 3816446A1 describe cooling circuits for a motor vehicles comprising a pump for ensuring the circulation of a cooling liquid such as water. GB2076471A describes a direct drive motor for cutting tools, flushing fluid is used as energizing medium and pumped down through the boring tube assembly. In its screw-like path through the motor, a portion of the pressure energy of the energizing medium is transformed into rotational energy for the shaft. WO8605555A1 discloses a screw-type rotary machine having at least one rotor made of a plastics material. US3461949A discloses apparatus for making threaded moulds.

### Summary

The invention is defined by a method of manufacturing a screw for a screw pump as in claim 1 and further by a screw pump as in claim 6.

According to a first aspect of the invention, a method of manufacturing a screw for a screw pump is provided, the method comprising molding a screw in a molding tool using a polymer material and ejecting the screw from the mold by applying an axial force to it, wherein the axial force causes the screw to rotate freely in the mold.

Further, according to another aspect of the invention, a screw pump is defined comprising a casing with an inlet, an outlet and a flow chamber between the inlet and the outlet; and at least two screws housed in the flow chamber to force a fluid flowthrough the flow chamber from the inlet to the outlet; wherein at least one of the screws is obtained by the method according to the first aspect of the invention and comprises threads with a helix angle of at least 60°.

The disclosure aims to improve the known designs of screw pumps and their performance.

The disclosure relates to a screw pump comprising: a casing with an inlet, an outlet and a flow chamber between the inlet and the outlet, and at least two screws housed in the flow chamber to force a fluid flow through the flow chamber from the inlet to the outlet, wherein at least one of the screws comprises a center shaft made of a first material on which the screw is molded from a second material.

For the sake of clarity, the first material and the second material can be materials that are distinct from each other or materials that are similar.

The first and second materials can be different. Advantageously, the first material is stiffer than the second material, for example such that the at least one screw is reinforced. The first material can comprise a metal, for example steel such as stainless steel, or a stiff polymer. The second material can comprise a polymer, wherein case the first material can comprise a polymer that is stiffer than the polymer of the second material. Thus, according to one aspect, the second material can be less stiff than the first material.

According to one aspect of the disclosure, at least one of the screws comprises a center shaft made of a first material on which the screw is molded from a second material that is less stiff than the first material.

As a variant, the first and second materials can be identical or similar. The first and second material can each comprise a polymer, for example a similar polymer.

The first and second materials can comprise one or more polymers. At least one of the polymers can comprise polyphenylene sulfide (PPS). The polymer, for example PPS, can be filled, for example with fibers such as glass fibers. The polymer, for example PPS, can be lubricated. The first and second materials can comprise the same polymer, for example PPS, which can be filled or additivated or neither differently for each of the first and second materials.

The center shaft can comprise one or more anchoring features or elements or members. The anchoring feature or features can be embedded in the second material, for example to anchor the center shaft in the second material.

The or each anchoring feature can comprise a rib or a spline, for example an axial rib or spline. The or each anchoring feature can extend along at least part of the center shaft.

The anchoring feature or features can comprise at least two anchoring features, or at least two groups of anchoring features, that can be spaced along the length of the center shaft.

The at least one reinforced screw can comprise a drive screw whose center shaft can comprise a motor coupling, for example to receive torque from a drive motor.

The casing can comprise a shell within which an insert defining the flow chamber is housed.

The disclosure also relates to a method of manufacturing a screw for a screw pump, the method comprising: providing a center shaft made of a first material, and molding a screw on the center shaft using a second material.

For the sake of clarity, the first material and the second material can be materials that are distinct from each other or materials that are similar.

The first and second materials can be different. The method can comprise: inserting the center shaft into the mold before the screw is molded on it. Advantageously, the first material is stiffer than the second material, for example such that the at least one screw is reinforced. The first material can comprise a metal or a stiff polymer.

Thus, according to one aspect, the second material can be less stiff than the first material.

As a variant, the first and second materials can be similar. The first and second materials can comprise a polymer. The method can comprise: molding the center shaft using the first material, for example before molding the screw on the center shaft using the second material. The method can comprise a two-step molding process.

The disclosure also relates to a screw pump comprising: a casing with an inlet, an outlet and a flow chamber between the inlet and the outlet, and at least two screws housed in the flow chamber to force a fluid flow through the flow chamber from the inlet to the outlet, wherein the casing comprises a shell within which an insert defining the flow chamber is housed.

The flow chamber can be defined by a tubular wall of the insert. The tubular wall can present, or can have, a substantially constant wall thickness. The tubular wall can have several cylindrical lobes, which can approximate the outer profile of the meshing screws. The cylindrical lobes can comprise a center lobe, for example which approximates the outer surfaces of a center drive screw. The cylindrical lobes can comprise an outer lobe on each side of the center lobe, for example which approximates the outer surfaces of a respective driven screw. The flow chamber can provide minimal space between the screws, while allowing them to rotate freely.

The casing can comprise a space between the shell and the insert. The interface between the shell and the insert can be designed to allow, when in use, part of the circulating fluid to enter the space. The space can be separate from the flow chamber and/or not be part of it.

The insert can comprise one or more anti-rotation protrusions, which can engage with the shell to inhibit relative rotation between them. The or each anti-rotation protrusion can extend axially from the insert. The or each anti-rotation protrusion can comprise an anti-rotation tab. The insert can comprise one or more anti-rotation protrusions extending from one or each of its ends. The insert can comprise a flange or clamp, such as a circular flange or clamp, at one end. The flange or clamp can have a perimeter which approximates an inner surface of the shell, for example to position the insert within the shell.

The pump can comprise a flexible coupling. The flexible coupling can be connected to one of the screws to couple the screw to a drive motor. The flexible coupling can be connected to the motor coupling of the center shaft of the drive screw.

The disclosure also relates to a screw pump comprising: a casing with an inlet, an outlet and a flow chamber between the inlet and the outlet, at least two screws housed in the flow chamber to force a fluid flow through the flow chamber from the inlet to the outlet, and a flexible coupling connected to one of the screws to couple the screw to a drive motor.

The flexible coupling can comprise a first side or end, for example with a first coupling feature to engage a shaft of a drive motor. The flexible coupling can comprise a second side or end, for example with a second coupling feature engaging a cooperating feature of the screw to which it is connected.

The first coupling feature can be a slot, which can be diametrical and/or which can be designed to house a protrusion on a shaft of the or of a drive motor. The second coupling feature can be a protrusion, for example to engage in a cooperating feature of the drive screw. The protrusion can be rectangular. The second coupling feature can be rotationally offset, for example by 90 degrees, relative to the first coupling feature.

The flexible coupling can comprise a polymer material, which can be lubricated, for example in its mass and/or by greasing.

At least one of the screws can be non-self-locking. The at least one screw can comprise one or more threads each having a pitch and/or a diameter and/or a configuration that enables it to be ejected from a mold by applying an axial force to it, for example without applying a rotational force to it. The at least one screw can comprise one or more threads each having a helix angle that enables them to be ejected from a mold by applying an axial force to it, for example without applying a rotational force to it.

The helix angle is at least 60°, for example at least 70°. The at least one screw can be made of a polymer, for example polyphenylene sulfide (PPS). The polymer, for example PPS, can be filled, for example with fibers such as glass fibers. The polymer, for example PPS, can be lubricated.

The disclosure also relates to a method of manufacturing a screw for a screw pump.

The disclosure also relates to a method of manufacturing a screw for a screw pump, the method comprising molding a screw in a molding tool using a polymer material and ejecting the screw from the mold by applying an axial force to it, wherein the material and threads of the screw are configured such that the axial force causes the screw to rotate freely in the mold.

The screw's self-locking can be inhibited by its configuration, in particular the helix angle of the thread or of each of the threads and/or the coefficient of friction between the thread or threads and the surfaces of the mold.

The disclosure also relates to a screw pump comprising: a casing with an inlet, an outlet and a flow chamber between the inlet and the outlet, and at least two screws housed in the flow chamber to force a fluid flow through the flow chamber from the inlet to the outlet, wherein at least one of the screws can be obtained by the method described above.

According to one aspect of the disclosure, at least one screw comprises a release coupling to restrict the rotation of the screw when it is being extracted from a molding tool.

According to one aspect of the disclosure, each screw comprises a release coupling to restrict the rotation of the screw when it is being extracted from a molding tool.

Of course, according to different variants of the disclosure, at least one or each screw, for example the or each self-locking screw, can comprise a release coupling. The release coupling can be used to restrict the rotation of the screw when it is being extracted from a molding tool.

The disclosure also relates to a screw pump comprising: a casing with an inlet, an outlet and a flow chamber between the inlet and the outlet, and at least two screws housed in the flow chamber to force a fluid flow through the flow chamber from the inlet to the outlet, wherein each screw comprises a release coupling to restrict the rotation of the screw when it is being extracted from a molding tool.

The insert can comprise at least one recess. The or each recess can be designed to house one of the release couplings, for example when the screws are housed in the flow chamber. One of the release couplings can be housed inside the recess.

In each of the aforementioned aspects of the disclosure, the screws can comprise three or more screws, or four or more screws. The screws can comprise at least one drive screw and at least one driven screw, for example at least two driven screws. Advantageously, the screws can comprise at least three driven screws, which can be distributed, for example evenly, around the drive screw.

The disclosure also relates to a method of manufacturing a screw for a screw pump, the method comprising molding a screw in a molding tool using a polymer material and ejecting the screw from the mold by applying torque to a screw release coupling while unscrewing the screw from the mold.

The release coupling of at least one of the screws can comprise at least one radial shoulder. The release coupling of at least one of the screws can comprise a circular or non-circular structure. The release coupling of at least one of the screws can comprise an annular or partially annular structure.

The disclosure also relates to a cooling circuit for a vehicle comprising a screw pump as described above.

For the avoidance of doubt, all the features described herein also apply to any aspect of the disclosure.

As part of this application, it is expressly provided that the various aspects, embodiments, examples and alternatives disclosed in the preceding paragraphs and/or in the following description and drawings, and in particular the individual features thereof, can be taken separately or in any combination. In other words, all aspects and/or features of any aspect can be combined in any way, unless these features are incompatible.

For the avoidance of doubt, the terms "can", "and/or", "for example", and any other similar term used herein must be interpreted as not limiting, such that any feature described herein is not necessarily required to be present. Indeed, any combination of optional features is expressly foreseen without departing from the scope of the disclosure.

### Brief Description Of The Figures

Other features and advantages of the disclosure will become apparent from the following detailed description, which will be understood in reference to the appended drawings, in which:
Figure 1 illustrates a pump assembly according to one aspect of the disclosure;
Figure 2 is an exploded view of the pump assembly in Figure 1;
Figure 3 illustrates a first side of the flow chamber insert of the pump assembly in Figures 1 and 2;
Figure 4 illustrates a second side of the flow chamber insert in Figure 3;
Figure 5 illustrates the insert in Figures 3 and 4 housed in the shell of the casing and with the screws housed in the insert;
Figure 6 illustrates the flexible coupling of the assembly in Figures 1 and 2, which couples the drive screw to the drive motor;
Figure 7 illustrates the drive screw of the pump assembly in Figures 1 and 2;
Figure 8 illustrates the center shaft of the drive screw in Figure 7;
Figure 9 illustrates a first side of the two driven screws of the pump assembly in Figures 1 and 2;
Figure 10 illustrates a second side of the driven screws in Figure 9;
Figure 11 illustrates a pump assembly according to another aspect of the disclosure;
Figure 12 illustrates a first side of the flow chamber insert of the pump assembly in Figure 11;
Figure 13 illustrates a second side of the flow chamber insert in Figure 12;
Figure 14 illustrates the insert in Figures 12 and 13 housed in the shell of the casing and with the screws housed in the insert;
Figure 15 illustrates the screw assembly of the pump assembly shown in Figure 11; and
Figure 16 illustrates another screw assembly that can be used in the pump assembly in Figure 11 instead of the screw assembly in Figure 15.

### Detailed Description

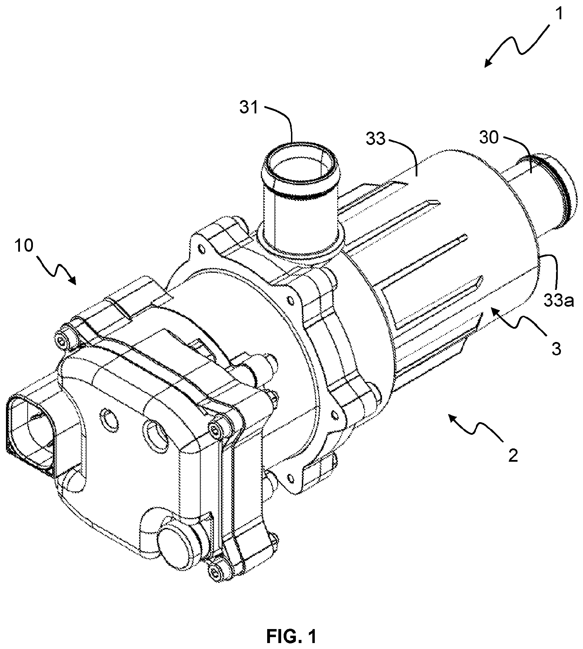

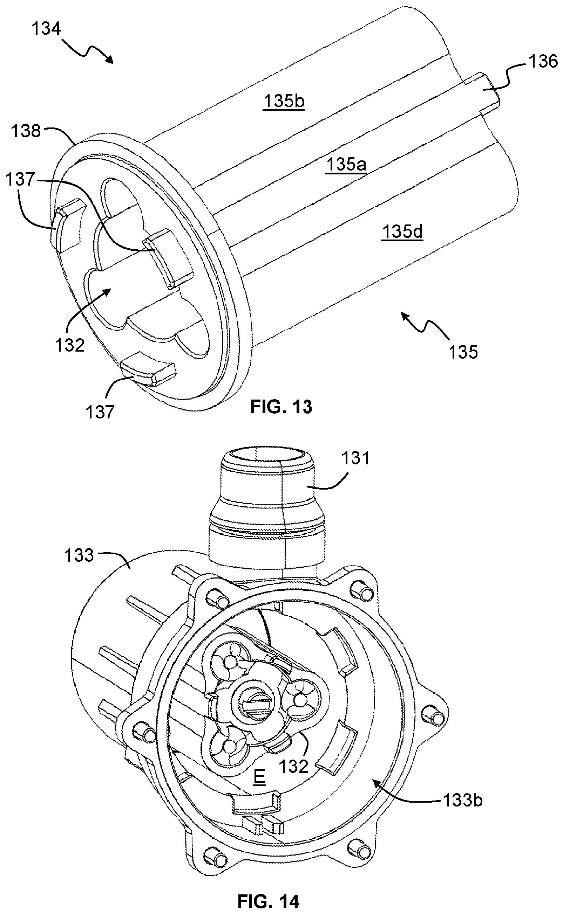

Different aspects of different aspects of the disclosure are described in more detail below, in reference to Figures 1 to 16 appended hereto.

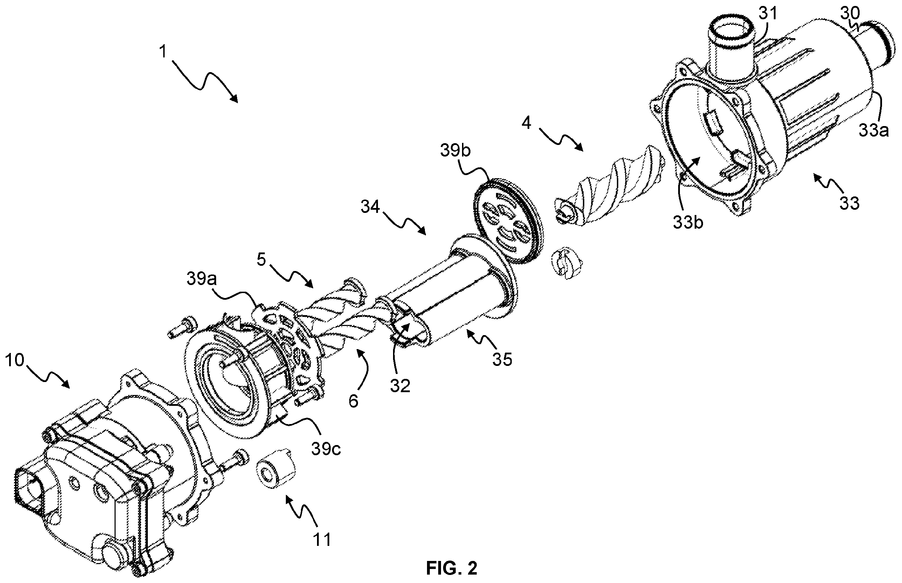

Referring now to Figures 1 and 2, a screw pump assembly 1 is shown, which comprises a motor 10 coupled to a screw pump 2 by a flexible coupling 11. The screw pump 2 comprises a casing 3 with an inlet pipe 30, an outlet pipe 31 and a flow chamber 32 between the inlet 30 and the outlet 31. Three screws 4, 5, 6 are housed in the flow chamber 32 to force fluid flow through the flow chamber 32 from the inlet 30 to the outlet 31.

The casing 3 comprises a shell 33 within which an insert 34 defining the flow chamber 32 is housed. The shell 33 is in the shape of a hollow cylinder with a closed end 33a from which the inlet pipe 30 protrudes. The outlet pipe 31 radially protrudes from the shell 33, next to an open end 33b. The screw pump 2 is reversible and, as such, the inlet pipe 30 and the outlet pipe 31 can be reversed by rotating the screw pump 2 in the opposite direction. However, for the sake of simplicity, the axial pipe 30 protruding from the closed end 33a will hereinafter be referred to as the inlet pipe 30 and the radial pipe 30 protruding from the open end 33b will hereinafter be referred to as the outlet pipe 31.

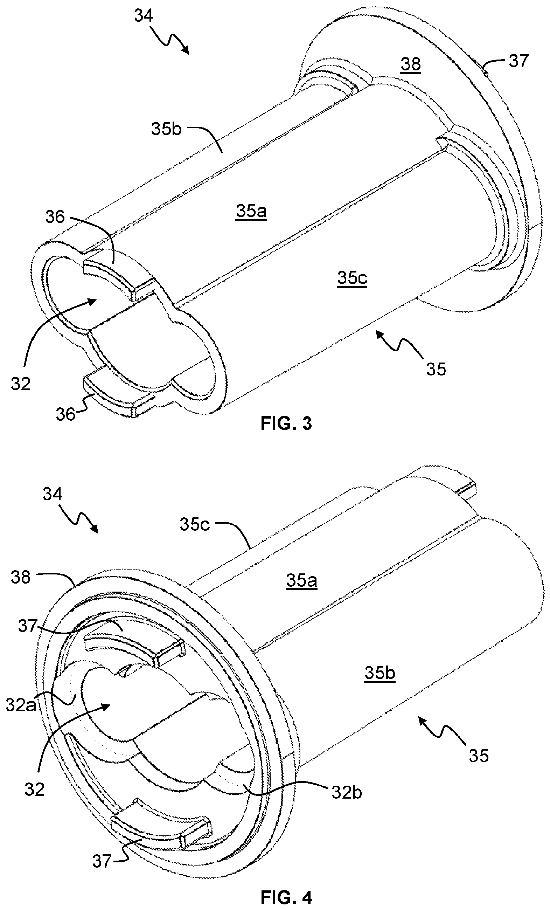

As shown in Figures 3 and 4, the flow chamber 32 is defined by a tubular wall 35 of the insert 34, which has a substantially constant wall thickness. The tubular wall 35 has three cylindrical lobes 35a, 35b, 35c which approximate the outer profile of the three meshing screws 4, 5, 6. More specifically, a center lobe 35a approximates the outer surfaces of a center drive screw 4, with an outer lobe 35b, 35c on each side of the center lobe 35a, each approximating the outer surfaces of a respective driven screw 5, 6. The flow chamber 32 provides minimal space between the screws 4, 5, 6, while allowing them to rotate freely.

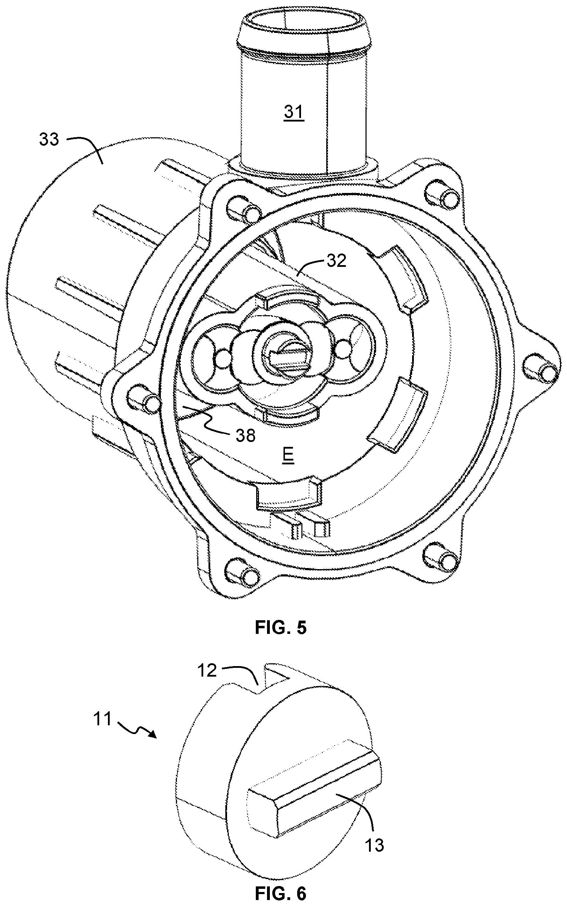

The insert 34 also comprises a pair of anti-rotation tabs 36, 37 protruding axially from each of its ends. A first pair of anti-rotation tabs 36 protrudes from the upper and lower parts of the center lobe 35a at a first end of the insert 34. A second pair of anti-rotation tabs 37 protrudes from a circular flange 38, above and below the center lobe 35a at a second end of the insert 34. The circular flange 38 has a perimeter which approximates an inner surface of the shell 33, which makes it possible to position the insert 34 within the shell 33 and to create a space E between them, as more clearly shown in Figure 5.

The casing 3 also comprises a pair of mounting discs 39a, 39b and a spacing interface 39c. The mounting discs 39a, 39b engage the anti-rotation tabs 36, 37 of the insert 34 and are attached inside the shell 33 to trap the screws 4, 5, 6 and the insert 34 between them. The spacing interface 39c sealingly closes the screw pump 2 and isolates the flow chamber 32 from the motor 10, but the interface between the shell 33 and the insert 34 is designed to allow, when in use, part of the circulating fluid to enter the space E.

In some examples, the first mounting disc 39a can be part of or integrated with the spacing interface 39c. In some examples, the second mounting disc 39b can be part of or integrated with the insert 34. When the second mounting disc 39b is part of the insert 34, the anti-rotation tabs 37 protruding from the circular flange 38 can be omitted.

The presence of a space E between the shell 33 and the insert 34, which is filled with circulating fluid, provides a vibration-damping effect resulting from the interaction between screws 4, 5, 6. In addition, the person skilled in the art will understand that the use of a separate insert 34 makes it possible to manufacture the flow chamber 32 with great precision.

This also makes it easier to manufacture the insert 34 by injection molding, since it can be designed with a substantially constant wall thickness to optimize cycle time and part quality.

The flexible coupling 11, more clearly illustrated in Figure 6, is substantially cylindrical and has a first coupling feature 12 at a first of its axial ends and a second coupling feature 13 at a second of its axial ends.

The first coupling feature 12 is a diametrical slot for designed to house a rectangular protrusion on a shaft of the drive motor 10. The second coupling feature 13 is a rectangular protrusion, which is rotationally offset by 90 degrees from the first coupling feature 12, to engage a cooperating feature of the drive screw 4.

In this example, the flexible coupling 11 is made of a lubricated polymer. The use of a flexible coupling between the drive motor 10 and the screw pump 2 makes it possible to accommodate minor angular and axial misalignment, while minimizing vibrations. The person skilled in the art will understand that this feature acts in synergy with the vibration-damping effect of the space E between the shell 33 and the insert 34.

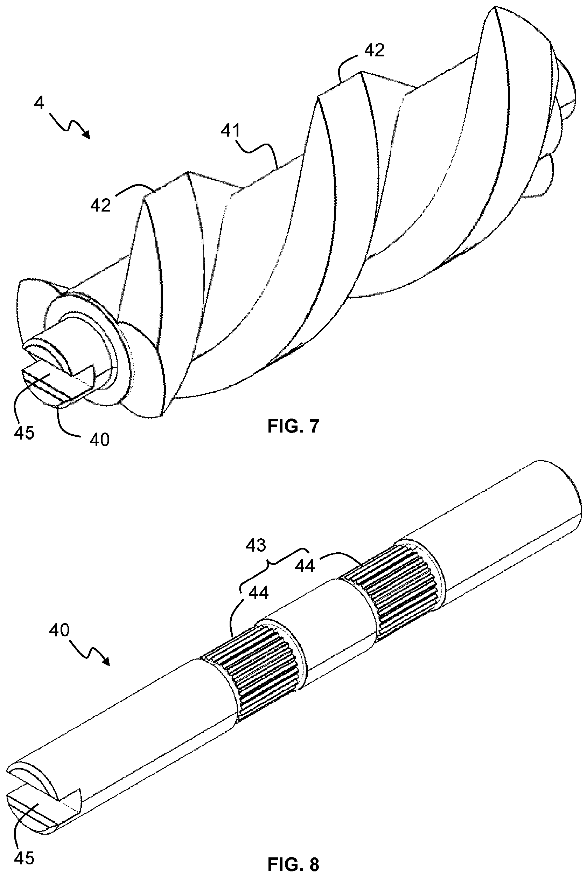

The drive screw 4 is more clearly shown in Figure 7, and comprises a center shaft 40 and a body 41 molded on the center shaft 40. In this example, the body 41 comprises two diametrically opposite threads 42 along its length.

The center shaft 40 comprises anchoring features 43 embedded in the body 41 to anchor the center shaft 40 to the body 41. In this example, the anchoring features 43 comprise two groups of axial splines 44 that extend along part of the center shaft 40. The two groups of axial splines 44 are spaced apart from one another along the length of the center shaft 40.

The center shaft 40 also comprises a motor coupling 45 in the form of a diametrical slot designed to house the rectangular protrusion 13 of the flexible coupling 11, although it can directly house the rectangular protrusion of the shaft of the drive motor 10. In this example, the center shaft 40 is made of stainless steel and the body 41 is made of a polymer material.

There are several advantages to using a polymer screw body 41 molded on a stainless-steel center shaft 40. The presence of the center shaft 40 reduces the thickness of the material required to form the body 41. The person skilled in the art will understand that this significantly reduces the cycle time and mitigates the tendency of the molded body 41 to deform when the material solidifies. In addition, the stiffness of the center shaft 40 also prevents the screw 4 from bending or deforming under load when torque is applied to it by the drive motor 10.

The person skilled in the art will also understand that this feature acts in synergy with the flexible coupling 11 and the vibration-damping effect of the space E between the shell 33 and the insert 34.

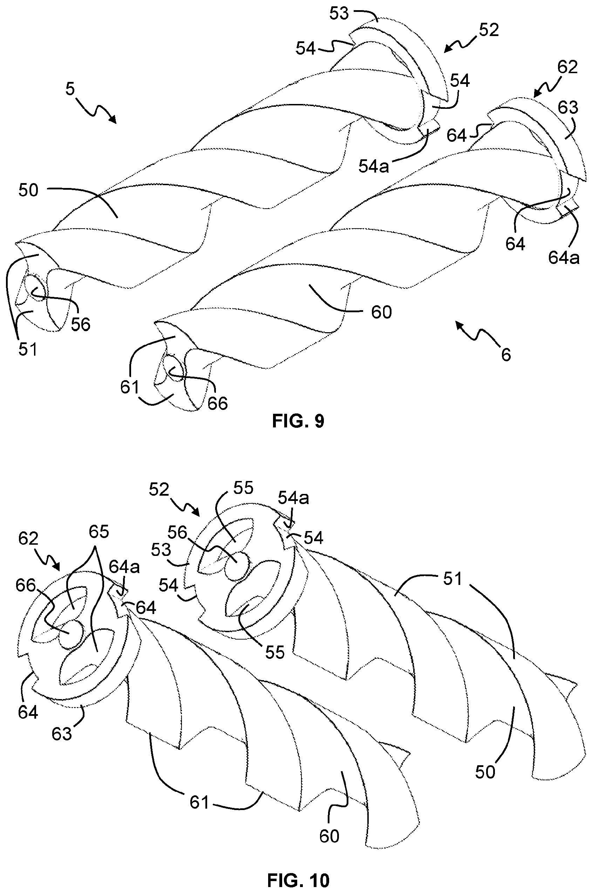

Figures 9 and 10 show the driven screws 5 and 6. Each driven screw 5, 6 comprises a respective body 50, 60 with a pair of diametrically opposite threads 51, 61 along its length. Each driven screw 5, 6 also comprises a release coupling 52, 62 at one of its ends. Each release coupling 52, 62 is in the shape of a ring 53, 63 with a pair of notches 54, 64 aligned with the adjacent ends of the threads 51, 61. The notches 54, 64 form radial shoulders 54a, 64a to which torque can be applied.

The diameter of the ring 53, 63 of each driven screw 5, 6 is larger than that of the threads 51, 61 and holes 55, 65 are defined between the ring 53, 63 and the base of the threads 51, 61. Thus, a fluid passage is defined along the entire length of each driven screw 5, 6, between the threads 51, 61 and through the release coupling 52, 62. Each driven screw 5, 6 also comprises an axial protrusion 56, 66 in the center of each of its ends.

In this example, the threads 51, 61 of the driven screws 5, 6 are self-locking, in that their rotation is prevented if only an axial force is applied to the driven screws 5, 6 at the end of the molding cycle, while they are still in the mold cavity (not shown). As such, torque must be applied to the driven screws 5, 6 to remove them from the mold. The release coupling 52, 62 allows this torque to be applied to the driven screws 5, 6.

In this example, the insert 34 comprises an annular step 32a, 32b surrounding the part of the flow chamber 32 defined by each of the outer lobes 35b, 35c. These annular steps 32a, 32b act as recesses that accommodate the rings 53, 63 when the screws 4, 5, 6 are housed in the flow chamber 32.

It will also be appreciated that the screw threads 51, 61 can alternatively be designed to be non-self-locking. In such circumstances, the release coupling 52, 62 can be omitted, and the driven screws 5, 6 can be ejected at the end of the molding process by simply applying axial force to them.

For example, the threads can each have a pitch and/or a diameter and/or a configuration that enables them to be ejected from a mold by applying an axial force to it, without applying a rotational force to it. More specifically, the threads can each have a helix angle that enables them to be ejected from a mold by applying an axial force to it, without applying a rotational force to it.

By way of example only, the helix angle can be at least 60°, for example at least 70°, when the threads are made of a polymer, such as fiberglass-filled polyphenylene sulfide (PPS).

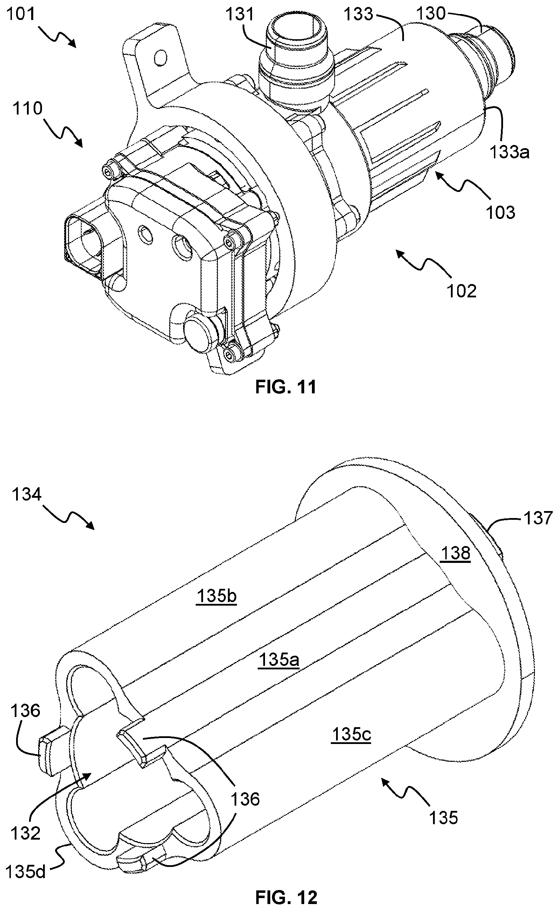

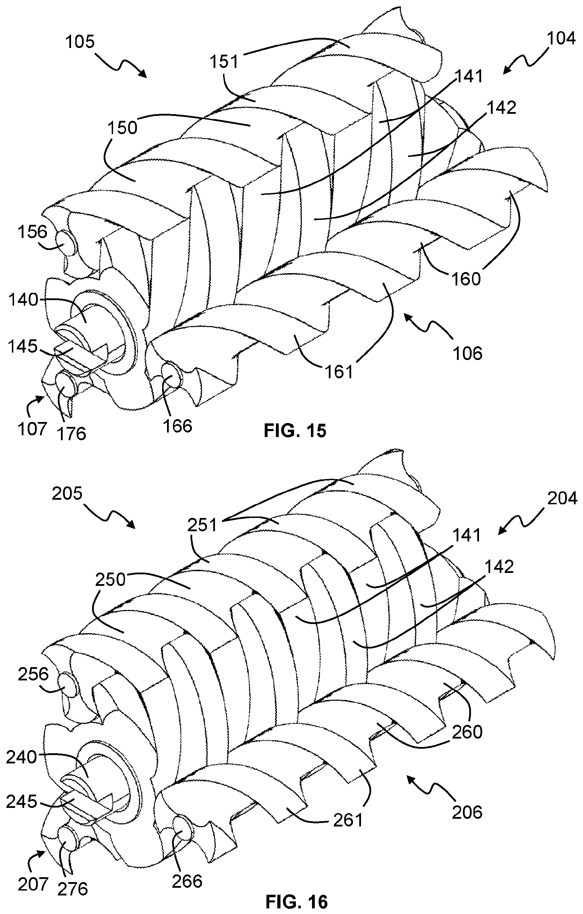

Referring now to figures 11 through 15, a screw pump assembly 101 according to a second example is shown, which is similar to the first example in that similar features are marked with like numbers incremented by 100. The screw pump assembly 1 in this example differs from that of the first example in that it comprises three driven screws 105, 106, 107 and that the drive screw has three threads 142, which is more clearly illustrated in Figure 15.

Therefore, the tubular wall 135 has four cylindrical lobes 135a, 135b, 135c, 135d, which approximate the outer profile of the four meshing screws 104, 105, 106, 107. More specifically, the center lobe 135a approximates the outer surfaces of the center drive screw 104, with three outer lobes 135b, 135c, 135d evenly distributed around the perimeter of the center lobe 135a, each approximating the outer surfaces of a respective driven screw 105, 106, 107.

Figure 16 shows another screw assembly 205, 206, 207 that can be used in the pump assembly in Figure 11 instead of the screw assembly in Figure 15. The screws 205, 206, 207 are similar to the ones in the previous example in that similar features are marked by like numbers incremented by 100. The screw assembly 205, 206, 207 in this example differs from the one in the previous example in that the helix angle is greater.

A person skilled in the art will be aware that several variants of the aforementioned aspects are conceivable without departing from the scope of the invention as defined by the appended claims.

Throughout the description and claims of this specification, the words "comprise" and "contain" and their variations mean "including but not limited to" and are not intended for (and do not exclude) other parts, additives, components, integers or steps.

Any features, integers, characteristics, compounds or groups described in connection with a particular aspect, embodiment or example of the disclosure are to be understood as being applicable to any other aspect, embodiment or example described herein, unless inconsistent therewith. All of the features disclosed in this specification (including the abstract and accompanying drawings), and/or all of the steps of a method or of a process thus disclosed, can be combined in any combination other than combinations wherein at least some of such features and/or steps are mutually exclusive. The disclosure is not limited to the details of all of the preceding aspects. The disclosure extends to any new feature or any new combination of features disclosed in this specification (including the abstract and accompanying drawings), or to any new feature, or any new combination, of the steps of any method or process thus disclosed.

### List of Reference Signs

- 1: screw pump assembly
- 10: motor
- 11: flexible coupling
- 12: first coupling feature
- 13: second coupling feature
- 2: screw pump
- 3: casing
- 30: inlet pipe
- 31: outlet pipe
- 32: flow chamber
- 32a: annular step
- 32b: annular step
- 33: shell
- 33a: closed end of shell
- 33b: open end of shell
- 34: insert
- 35: tubular insert wall
- 35a: cylindrical center lobe of the tubular wall
- 35b: cylindrical outer lobe of the tubular wall
- 35c: cylindrical outer lobe of the tubular wall
- 36: anti-rotation tabs
- 37: anti-rotation tabs
- 38: circular flange
- 39a: mounting disc
- 39b: mounting disc
- 39c: spacing interface
- 4: drive screw
- 40: drive screw center shaft
- 41: drive screw body
- 43: anchoring features
- 44: axial splines
- 45: motor coupling
- 5: driven screw
- 50: driven screw body
- 51: driven screw threads
- 52: release coupling
- 53: release coupling ring
- 54: release coupling notch
- 54a: radial shoulder
- 55: hole
- 56: axial protrusion
- 6: driven screw
- 60: driven screw body
- 61: driven screw threads
- 62: release coupling
- 63: release coupling ring
- 64: release coupling notch
- 64a: radial shoulder
- 65: hole
- 66: axial protrusion
- E: space between insert and shell
- 101: screw pump assembly
- 110: motor
- 102: screw pump
- 103: casing
- 130: inlet pipe
- 131: outlet pipe
- 132: flow chamber
- 133: shell
- 133a: closed end of shell
- 133b: open end of shell
- 134: insert
- 135: tubular insert wall
- 135a: cylindrical center lobe of the tubular wall
- 135b: cylindrical outer lobe of the tubular wall
- 135c: cylindrical outer lobe of the tubular wall
- 135d: cylindrical outer lobe of the tubular wall
- 136: anti-rotation tabs
- 137: anti-rotation tabs
- 138: circular flange
- 104: drive screw
- 140: drive screw center shaft
- 141: drive screw body
- 145: motor coupling
- 105: driven screw
- 150: driven screw body
- 151: driven screw threads
- 156: axial protrusion
- 106: driven screw
- 160: driven screw body
- 161: driven screw threads
- 166: axial protrusion
- 107: driven screw
- 176: axial protrusion
- 204: drive screw
- 240: drive screw center shaft
- 241: drive screw body
- 245: motor coupling
- 205: driven screw
- 250: driven screw body
- 251: driven screw threads
- 256: axial protrusion
- 206: driven screw
- 260: driven screw body
- 261: driven screw threads
- 266: axial protrusion
- 207: driven screw
- 276: axial protrusion

## Claims

1. A method of manufacturing a screw for a screw pump, the method comprising molding a screw in a molding tool using a polymer material and ejecting the screw from the mold by applying an axial force to it, wherein the axial force causes the screw to rotate freely in the mold.

2. The method according to claim 1, wherein the screw's self-locking is inhibited by the helix angle of the threads and the coefficient of friction between the threads and the surfaces of the mold.

3. The method according to claim 1 or claim 2, wherein the helix angle of the threads is at least 60°.

4. The method according to any one of the preceding claims, wherein the helix angle of the threads is at least 70°.

5. The method according to any one of the preceding claims, wherein the polymer material comprises polyphenylene sulfide.

6. A screw pump (2) comprising:
a casing (3) with an inlet (30), an outlet (31) and a flow chamber (32) between the inlet and the outlet; and
at least two screws (4, 5, 6) housed in the flow chamber to force a fluid flow through the flow chamber from the inlet to the outlet;
wherein at least one of the screws is obtained by a method according to any one of the preceding claims and comprises threads with a helix angle of at least 60°.

7. The screw pump according to claim 6, wherein at least one of the screws comprises threads with a helix angle of at least 70°.

8. The screw pump according to claim 6 or claim 7, wherein at least one of the screws comprises a polymer material comprising polyphenylene sulfide.

9. The screw pump according to any one of claims 6 to 8, wherein at least one of the screws (4) comprises a center shaft (40) made of a first material on which the screw is molded from a second material that is less stiff than the first material.

10. The screw pump according to any one of claims 6 to 9, wherein the casing comprises a shell (33) within which an insert (34) defining the flow chamber is housed.

11. The screw pump according to claim 10, wherein the flow chamber is defined by a tubular wall (35) of the insert that has a substantially constant wall thickness.

12. The screw pump according to claim 10 or claim 11, wherein the insert comprises one or more anti-rotation protrusions (36, 37) which engage(s) with the shell to inhibit relative rotation between them.

13. The screw pump according to any one of claims 6 to 12, comprising a flexible coupling (11) connected to one of the screws (4) to couple the screw to a drive motor.

14. A pump assembly (1) comprising a pump according to claim 13 and a drive motor (10) coupled to the flexible coupling to drive the screws so as to force a fluid flow through the flow chamber from the inlet to the outlet.

## Patentansprüche

1. Verfahren zur Herstellung einer Schnecke für eine Schraubenpumpe, wobei das Verfahren das Formen einer Schnecke in einem Formwerkzeug unter Verwendung eines Polymermaterials und das Auswerfen der Schnecke aus der Form durch Aufbringen einer axialen Kraft auf diese aufweist, wobei die axiale Kraft bewirkt, dass sich die Schnecke frei in der Form dreht.

2. Verfahren nach Anspruch 1, wobei die Selbsthemmung der Schnecke durch den Schrägungswinkel der Gewinde und den Reibungskoeffizienten zwischen den Gewinden und den Oberflächen der Form verhindert wird.

3. Verfahren nach Anspruch 1 oder Anspruch 2, wobei der Schrägungswinkel der Gewinde mindestens 60° beträgt.

4. Verfahren nach einem der vorhergehenden Ansprüche, wobei der Schrägungswinkel der Gewinde mindestens 70° beträgt.

5. Verfahren nach einem der vorhergehenden Ansprüche, wobei das Polymermaterial Polyphenylensulfid aufweist.

6. Schraubenpumpe (2), aufweisend:
ein Gehäuse (3) mit einem Einlass (30), einem Auslass (31) und einer Durchflusskammer (32) zwischen dem Einlass und dem Auslass; und
mindestens zwei Schnecken (4, 5, 6), die so in der Durchflusskammer untergebracht sind, dass sie einen Fluidstrom durch die Durchflusskammer von dem Einlass zu dem Auslass leiten;
wobei mindestens eine der Schnecken durch ein Verfahren nach einem der vorhergehenden Ansprüche erhalten wird und Gewinde mit einem Schrägungswinkel von mindestens 60° aufweist.

7. Schraubenpumpe nach Anspruch 6, wobei mindestens eine der Schnecken Gewinde mit einem Schrägungswinkel von mindestens 70° aufweist.

8. Schraubenpumpe nach Anspruch 6 oder Anspruch 7, wobei mindestens eine der Schnecken ein Polymermaterial aufweist, das Polyphenylensulfid aufweist.

9. Schraubenpumpe nach einem der Ansprüche 6 bis 8, wobei mindestens eine der Schnecken (4) eine Mittelwelle (40) aufweist, die aus einem ersten Material hergestellt ist, auf das die Schnecke aus einem zweiten Material, das weniger steif als das erste Material ist, aufgeformt wird.

10. Schraubenpumpe nach einem der Ansprüche 6 bis 9, wobei das Gehäuse eine Schale (33) aufweist, in der ein Einsatz (34) untergebracht ist, der die Strömungskammer definiert.

11. Schraubenpumpe nach Anspruch 10, wobei die Durchflusskammer durch eine rohrförmige Wand (35) des Einsatzes, die eine im Wesentlichen konstante Wanddicke aufweist, definiert ist.

12. Schraubenpumpe nach Anspruch 10 oder Anspruch 11, wobei der Einsatz einen oder mehrere Verdrehsicherungsvorsprünge (36, 37) aufweist, die mit der Schale in Eingriff stehen, um eine relative Drehung zwischen ihnen zu verhindern.

13. Schraubenpumpe nach einem der Ansprüche 6 bis 12, aufweisend eine flexible Kupplung (11), die so mit einer der Schnecken (4) verbunden ist, dass sie die Schnecke mit einem Antriebsmotor koppelt.

14. Pumpenanordnung (1), aufweisend eine Pumpe nach Anspruch 13 und einen Antriebsmotor (10), der so mit der flexiblen Kupplung gekoppelt ist, dass er die Schnecken antreibt und einen Fluidstrom durch die Strömungskammer von dem Einlass zu dem Auslass leitet.

## Revendications

1. Procédé de fabrication d'une vis pour une pompe à vis, le procédé comprenant le moulage d'une vis dans un outil de moulage en utilisant un matériau polymère et l'éjection de la vis du moule en lui appliquant une force axiale, dans lequel la force axiale amène la vis à tourner librement dans le moule.

2. Procédé selon la revendication 1, dans lequel l'autoblocage de la vis est empêché par l'angle d'hélice des filets et le coefficient de frottement entre les filets et les surfaces du moule.

3. Procédé selon la revendication 1 ou la revendication 2, dans lequel l'angle d'hélice des filets est d'au moins 60°.

4. Procédé selon l'une quelconque des revendications précédentes, dans lequel l'angle d'hélice des filets est d'au moins 70°.

5. Procédé selon l'une quelconque des revendications précédentes, dans lequel le matériau polymère comprend du sulfure de polyphénylène.

6. Pompe à vis (2) comprenant :
un boîtier (3) avec une entrée (30), une sortie (31) et une chambre d'écoulement (32) entre l'entrée et la sortie ; et
au moins deux vis (4, 5, 6) logées dans la chambre d'écoulement pour forcer un écoulement de fluide à travers la chambre d'écoulement depuis l'entrée jusqu'à la sortie ;
dans lequel au moins une des vis est obtenue selon un procédé de l'une quelconque des revendications précédentes et comprend des filets avec un angle d'hélice d'au moins 60°.

7. Pompe à vis selon la revendication 6, dans laquelle au moins une des vis comprend des filets avec un angle d'hélice d'au moins 70°.

8. Pompe à vis selon la revendication 6 ou la revendication 7, dans laquelle au moins une des vis comprend un matériau polymère comprenant du sulfure de polyphénylène.

9. Pompe à vis selon l'une quelconque des revendications 6 à 8, dans laquelle au moins une des vis (4) comprend un arbre central (40) composé d'un premier matériau sur lequel la vis est moulée à partir d'un deuxième matériau qui est moins rigide que le premier matériau.

10. Pompe à vis selon l'une quelconque des revendications 6 à 9, dans laquelle le boîtier comprend une enveloppe (33) au sein de laquelle est logé un insert (34) définissant la chambre d'écoulement.

11. Pompe à vis selon la revendication 10, dans laquelle la chambre d'écoulement est définie par une paroi tubulaire (35) de l'insert qui a une épaisseur de paroi sensiblement constante.

12. Pompe à vis selon la revendication 10 ou la revendication 11, dans laquelle l'insert comprend une ou plusieurs saillies anti-rotation (36, 37) qui viennent en prise avec l'enveloppe pour empêcher une rotation relative entre elles.

13. Pompe à vis selon l'une quelconque des revendications 6 à 12, comprenant un couplage flexible (11) relié à l'une des vis (4) pour coupler la vis à un moteur d'entraînement.

14. Ensemble de pompe (1) comprenant une pompe selon la revendication 13 et un moteur d'entraînement (10) couplé au couplage flexible pour entraîner les vis de manière à forcer un écoulement de fluide à travers la chambre d'écoulement depuis l'entrée jusqu'à la sortie.
